(19) Europäisches Patentamt
European Patent Office
Office européen des brevets

(11) Veröffentlichungsnummer: **0 189 034**
**B1**

(12) # EUROPÄISCHE PATENTSCHRIFT

(45) Veröffentlichungstag der Patentschrift: **01.03.89**

(51) Int. Cl.⁴: **C 07 D 249/08**, C 07 D 233/60, A 01 N 43/64, A 01 N 43/50

(21) Anmeldenummer: **86100050.3**

(22) Anmeldetag: **03.01.86**

(54) **Azolyl-aroxymethyl-dimethylpentinole.**

(30) Priorität: **16.01.85 DE 3501245**

(43) Veröffentlichungstag der Anmeldung: **30.07.86 Patentblatt 86/31**

(45) Bekanntmachung des Hinweises auf die Patenterteilung: **01.03.89 Patentblatt 89/9**

(84) Benannte Vertragsstaaten: **AT BE CH DE FR GB IT LI NL SE**

(56) Entgegenhaltungen:
**EP-A- 0 073 331**
**EP-A- 0 110 048**
**EP-A- 0 112 292**
**EP-A- 0 123 160**
**EP-A- 0 129 798**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Jäger, Gerhard, Dr., Paul-Klee-Strasse 28j, D-5090 Leverkusen (DE)**
Erfinder: **Jautelat, Manfred, Dr., Müllersbaum 28, D-5093 Burscheid (DE)**
Erfinder: **Arlt, Dieter, Prof.Dr., Rybniker Strasse 2, D-5000 Köln 80 (DE)**
Erfinder: **Reinecke, Paul, Dr., Steinstrasse 8, D-5090 Leverkusen (DE)**
Erfinder: **Brandes, Wilhelm, Dr., Eichendorffstrasse 3, D-5653 Leichlingen (DE)**
Erfinder: **Hänssler, Gerd, Dr., Am Arenzberg 58a, D-5090 Leverkusen (DE)**

**EP 0 189 034 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Azolyl-aroxymethyl-dimethylpentinole, ein Verfahren zu deren Herstellung und deren Verwendung als Fungizide.

Aus der EP-A-0 084 834 sind bereits substituierte l-Hydroxyalkyl-azolyl-Derivate, in denen das Carbinol-Kohlenstoffatom einen substituierten, verzweigten Alkylrest trägt, mit fungiziden Eigenschaften bekannt geworden. Verbindungen dieses Typs, in denen der Substituent am verzweigten Alkylrest für Alkinyl steht, werden jedoch nicht beschrieben. Außerdem ist die Wirkung der offenbarten Stoffe, insbesondere bei niedrigen Anwendungskonzentrationen, nicht immer voll befriedigend.

Ferner sind aus der US-A-4 578 396 fungizid wirksame Hydroxyethyl-azole bekannt, in denen das Carbinol-Kohlenstoffatom mit einer Propargyl-Gruppe verbunden ist. Entsprechende Verbindungen, in denen das Kohlenstoffatom in Nachbarstellung zum Carbinol-Kohlenstoffatom in Nachbarstellung zum Carbinol-Kohlenstoff noch zwei Methylgruppen trägt, werden aber nicht erwähnt.

Es wurden nun die neuen Azolyl-aroxymethyl-dimethylpentinole der allgemeinen Formel (I)

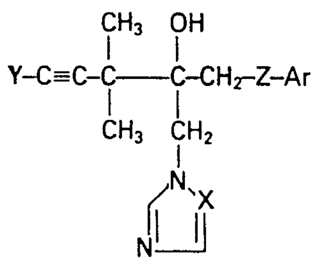

(I)

in welcher

X für ein Stickstoffatom oder die CH-Gruppe steht,

Y für ein Wasserstoff-, Brom- oder Jodatom steht,

Z für ein Sauerstoff- oder Schwefelatom, die $-SO-$ oder die $-SO_2$-Gruppe steht, und

Ar für Phenyl oder für Naphthyl steht, wobei die genannten Reste substituiert sein können durch Halogen, durch Alkyl, Alkoxy und Alkylthio mit jeweils bis zu 4 Kohlenstoffatomen, durch Halogenalkyl, Halogenalkoxy und Halogenalkylthio mit jeweils bis zu 2 Kohlenstoff- und bis zu 5 Halogenatomen, durch gegebenenfalls halogensubstituiertes Phenyl oder Phenoxy und schließlich durch die Gruppen $CH_3-O-N=CH-$ und $CH_3-O-N=C(CH_3)-$, sowie deren pflanzenphysiologisch verträgliche Säureadditions-Salze und Metallsalz-Komplexe gefunden.

Weiterhin wurde gefunden, daß man die neuen Azolyl-aroxymethyl-dimethyl-pentinole der allgemeinen Formel (I) sowie deren pflanzenphysiologisch verträgliche Säureadditions-Salze und Metallsalz-Komplexe erhält, wenn man Oxiran-Derivate der allgemeinen Formel (II)

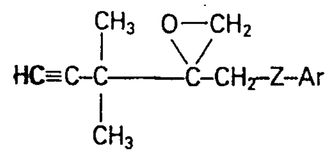

(II)

in welcher Z und Ar die obengenannte Bedeutung besitzen, mit Azolen der allgemeinen Formel (III)

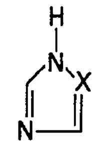

(III)

in welcher X die obengenannte Bedeutung hat, gegebenenfalls in Gegenwart eines Verdünnungsmittels und einer Base zur Umsetzung bringt, und gegebenenfalls die dabei erhaltenen Verbindungen der allgemeinen Formel (Ia)

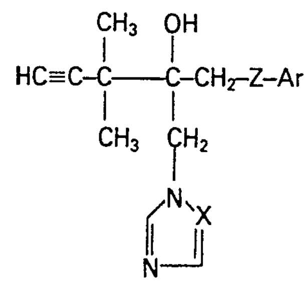

(Ia)

in welcher X, Z und Ar die obengenannte Bedeutung besitzen, mit Brom oder Jod, gegebenenfalls in Gegenwart eines Verdünnungsmittels und eines Säurebindemittels, halogeniert, und weiterhin gegebenenfalls noch an die so erhaltenen Verbindungen der allgemeinen Formel (I) eine Säure oder ein Metallsalz addiert.

Die neuen Azolyl-aroxymethyl-dimethylpentinole der allgemeinen Formel (I) sowie deren pflanzenphysiologisch verträgliche Säureadditions-Salze und Metallsalz-Komplexe weisen starke fungizide Eigenschaften auf. Überraschenderweise zeigen dabei die erfindungsgemäßen Verbindungen eine erheblich höhere fungizide Wirkung als die aus dem Stand der Technik bekannten l-Hydroxyalkylazolyl-Derivate. Die neuen Verbindungen stellen daher eine Bereicherung des Standes der Technik dar.

Bevorzugt sind diejenigen Verbindungen der allgemeinen Formel (I), in denen

Ar für Phenyl steht, welches gegebenenfalls ein- bis dreifach durch Fluor und Chlor, durch Methyl, Ethyl und Isopropyl, durch Halogenmethyl mit 1 bis 3 Fluor- oder Chloratomen, durch Methoxy, Methylthio, Trifluormethoxy und Trifluormethylthio und/oder durch die Gruppen $CH_3-O-N=CH-$ und $CH_3-O-N=C(CH_3)-$, sowie einfach durch Phenyl oder Phenoxy substituiert sein kann, wobei die beiden letztgenannten Reste ihrerseits gegebenenfalls ein- bis dreifach durch Fluor und Chlor substituiert sein können, und X, Y und Z die in der Erfindungsdefinition angegebene Bedeutung besitzen.

Im einzelnen seien beispielhaft – neben den bei den Herstellungsbeispielen genannten Verbindungen – die folgenden Verbindungen der allgemeinen Formel (I) genannt:

| X | Y | Z | Ar |
|---|---|---|---|
| CH | H | O | 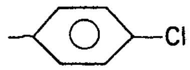 |
| CH | J | O | 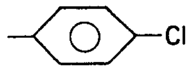 |
| CH | H | O | 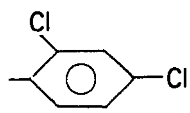 |
| CH | J | O | 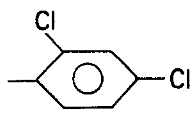 |
| CH | H | O | 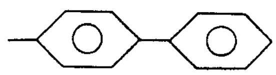 |
| CH | J | O | 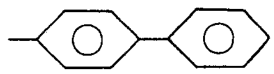 |
| CH | Br | O | 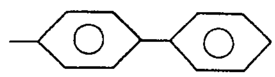 |
| CH | H | O | 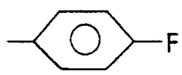 |
| CH | I | O | 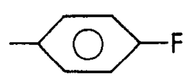 |
| CH | H | S | 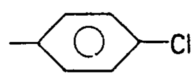 |
| CH | I | S | 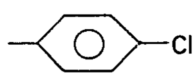 |
| N | H | S | 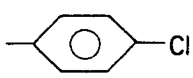 |
| N | I | S | 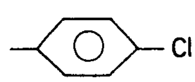 |

| X | Y | Z | Ar |
|---|---|---|---|
| N | H | S | 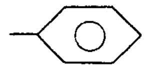 |
| N | I | S | 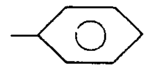 |
| N | H | SO | 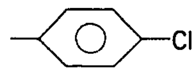 |
| N | H | $SO_2$ | 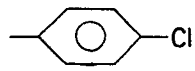 |
| N | I | $SO_2$ | 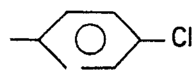 |
| N | I | O | 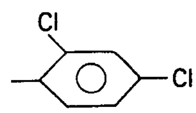 |
| N | Br | O | 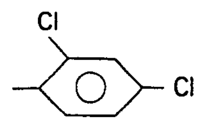 |
| N | Br | O | 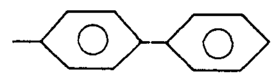 |
| N | I | O | 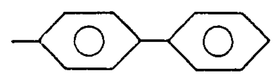 |
| N | H | O | 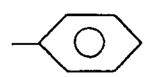 |
| N | I | O | 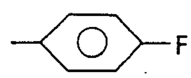 |
| N | H | O | 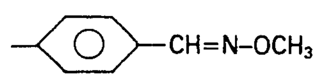 |
| N | I | O | 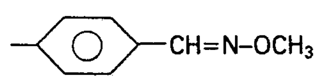 |
| N | H | O | 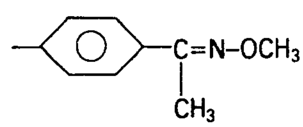 |

| X | Y | Z | Ar |
|---|---|---|---|
| N | H | O | 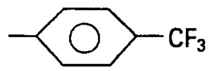 |
| N | I | O | 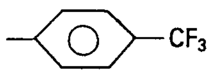 |
| N | H | O | 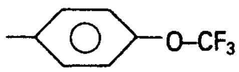 |
| N | H | O | 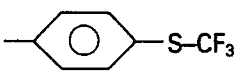 |
| N | H | O | 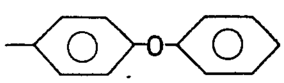 |

Verwendet man zur Herstellung der erfindungsgemäßen Verbindungen der allgemeinen Formel (I) beispielsweise 2-(4-Chlorphenoxymethyl)-2-(3-methyl-1-butin-3-yl)-oxiran und 1, 2, 4-Triazol in Gegenwart von Natrium-butanolat als Ausgangsstoffe, so kann der Reaktionsablauf durch das folgende Formelschema wiedergegeben werden:

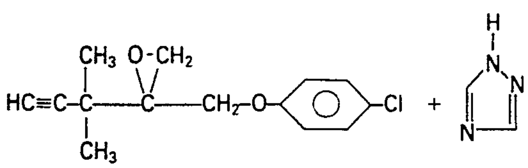

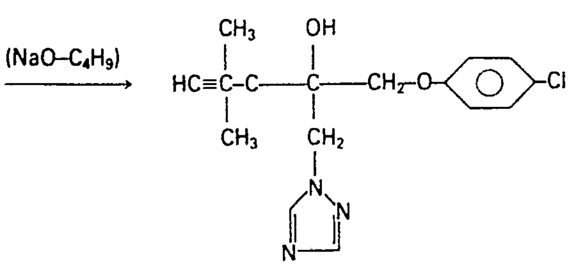


Halogeniert man die so erhaltene Verbindung beispielsweise mit Jod in Gegenwart von Natronlauge, so läßt sich dies wie folgt wiedergeben:

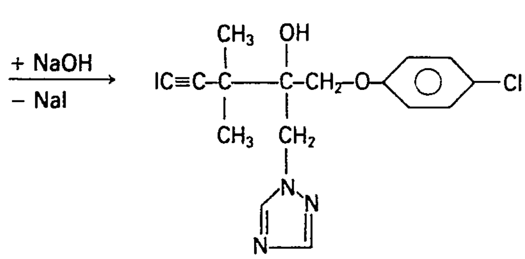


Die als Ausgangsstoffe zu verwendenden Oxiran-Derivate sind durch die allgemeine Formel (II) definiert. In dieser Formel haben Z und Ar vorzugsweise die Bedeutungen, die schon bei Besprechung der Verbindungen der allgemeinen Formel (I) vorzugsweise für diese Substituenten genannt wurden.

Die Oxiran-Derivate der allgemeinen Formel (II) lassen sich nach allgemein bekannten Methoden herstellen (siehe hierzu EP-A 0-084 834). So lassen sich beispielsweise die entsprechenden Ketone der allgemeinen Formel (IV)

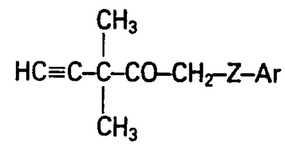 (IV)

in welcher
Z und Ar die oben angegebene Bedeutung besitzen, entweder (a) mit Dimethyloxosulfonium-methylid in Gegenwart eines geeigneten Verdünnungsmittels, wie z.B. Dimethylsulfoxid, im Temperaturbereich zwischen +20 und 80 °C umsetzen, oder aber (b) mit Trimethylsulfonium-methylsulfat in Gegenwart eines inerten organischen Lösungsmittels, wie z.B. Acetonitril, und in Gegenwart einer Base, wie z.B. Natrium-methylat, im Temperaturbereich zwischen 0 und 60 °C zur Reaktion bringen [vgl. hierzu die zitierte EP-A-0 084 834 und die Angaben in J. Amer. Chem. Soc. 87, Seite 1363 – 1364 (1965) mit weiteren Einzelheiten].

Die Ketone der allgemeinen Formel (IV) werden ebenfalls in bekannter Weise erhalten, indem man 1-Chlor-3,3-dimethyl-4-pentin-2-on der Formel (V)

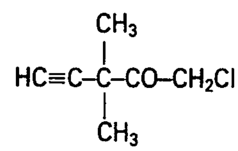 (V)

mit Phenolen oder Thiophenolen in Gegenwart eines Säurebindemittels, wie Kaliumcarbonat, und eines Verdünnungsmittels wie Aceton, im Temperaturbereich zwischen +40 und 100 °C umsetzt (bekannte Ether-Synthese nach Williamson). Die dabei erhaltenen Verbindungen der allgemeinen Formel (VI)

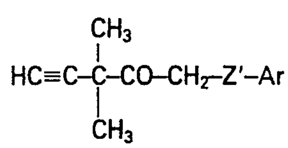 (VI)

in welcher
Z' für Sauerstoff oder Schwefel steht und
Ar die weiter oben angegebenen Bedeutungen besitzt,
können im Falle der Schwefel-Verbindungen noch in die Stufe der Sulfoxide und Sulfone übergeführt werden. Man arbeitet dabei in allgemein üblicher Weise und führt die Oxidation beispielsweise mit Benzoylperoxid oder Wasserstoffperoxid bzw. mit Kaliumpermanganat (letzteres nur für die Sulfonstufe) durch.

Die zur Herstellung der erfindungsgemäßen Verbindungen der allgemeinen Formel (I) weiterhin benötigten Azole sind durch die allgemeine Formel (III) eindeutig definiert. Die von dieser Formel umfaßten Verbindungen Imidazol und 1, 2, 4-Triazol sind allgemein bekannte, laboratoriumsübliche Verbindungen.

Als Verdünnungsmittel kommen zur Herstellung der erfindungsgemäßen Verbindungen inerte organische Lösungsmittel in Frage. Hierzu gehören vorzugsweise Alkohole, wie Methanol, Ethanol, Isopropanol oder Butanol, ferner Nitrile, wie Acetonitril, weiterhin Ether, wie Tetrahyrofuran oder Dioxan, und schließlich Dimethylformamid.

Die Herstellung der erfindungsgemäßen Verbindungen wird vorzugsweise in Gegenwart von Basen durchgeführt. Hierfür kommen alle üblicherweise verwendbaren anorganischen und organischen Basen in Betracht. Hierzu gehören vorzugsweise Alkalicarbonate, wie z. B. Natrium- und Kaliumcarbonat, Alkalihydroxide, wie z. B. Natriumhydroxid, Alkalialkoholate, wie z. B. Natrium- und Kalium-methylat, -ethylat, und -butylat, Alkalihydride, wie z. B. Natriumhydrid, und schließlich tertiäre Amine, wie Triethylamin.

Die Reaktionstemperaturen können bei der Durchführung der Herstellung in einem größeren Bereich variiert werden. Im allgemeinen arbeitet man zwischen +40 und 180 °C.

Bei der Durchführung des Herstellungsverfahrens setzt man vorzugsweise auf 1 Mol Oxiran der allgemeinen Formel (II) 1 bis 2 Mol Azol der allgemeinen Formel (III) und gegebenenfalls bis zu 1,5 Mol Base ein. Die Isolierung der Endprodukte erfolgt in allgemein üblicher Weise.

Die nach den erfindungsgemäßen Verfahren erhältlichen Verbindungen der allgemeinen Formel (I) können in üblicher Weise in Säureadditions-Salze bzw. Metallsalz-Komplexe überführt werden (man vergleiche hierzu auch die Angaben in der bereits zitierten EP-A-0 084 834).

Zur Herstellung von physiologisch verträglichen Säureadditionssalzen der Verbindungen der allgemeinen Formel (I) kommen vorzugsweise folgende Säuren in Frage: Die Halogenwasserstoffsäuren, wie z. B. Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z. B. Essigsäure, Maleinsäure, Bernsteinsäure, Fumarsäure, Weinsäure, Citronensäure, Salicylsäure, Sorbinsäure, Milchsäure sowie Sulfonsäuren wie z. B. p-Toluolsulfonsäure und 1,5-Naphthalindisulfonsäure.

Zur Herstellung von Metallsalz-Komplexen der Verbindungen der allgemeinen Formel (I) kommen vorzugsweise Salze von Metallen der II. bis IV. Hauptgruppe und der I. und II. sowie IV. bis VIII. Nebengruppe des Periodensystems in Frage, wobei Kupfer, Zinn, Eisen und Nickel beispielhaft genannt seien. Als Anionen der Salze kommen solche in Betracht, die sich vorzugsweise von folgenden Säuren ableiten: Halogenwasserstoffsäure, wie z. B. die Chlorwasserstoffsäure und die Bromwasserstoffsäure, ferner Phosphorsäure, Salpetersäure und Schwefelsäure.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft, aber nicht begrenzend, seien einige Erreger von pilzlichen und bakteriellen Erkrankungen, die unter die oben aufgezählten Oberbegriffe fallen, genannt:
Uromyces-Arten, wie beispielsweise Uromyces appendiculatus;
Sphaerotheca-Arten, wie beispielsweise Sphaerotheca fuliginea;
Venturia-Arten, wie beispielsweise Venturia inaequalis;
Podosphaera-Arten, wie beispielsweise Podosphaera leucotricha;
Phytophthora-Arten, wie beispielsweise Phytophthora infestans;
Erysiphe-Arten, wie beispielsweise Erysiphe graminis;
Puccinia-Arten, wie beispielsweise Puccinia recondita;
Drechslera-Arten, wie beispielsweise Drechslera graminea (Synonym: Helminthosporium);
Fusarium-Arten, wie beispielsweise Fusarium culmorum;
Ustilago-Arten, wie beispielsweise Ustilago nuda oder Ustilago avenae;
Septoria-Arten, wie beispielsweise Septoria nodorum;
Tilletia-Arten, wie beispielsweise Tilletia caries;
Xanthomonas-Arten, wie beispielsweise Xanthomonas oryzae;
Pseudomonas-Arten, wie beispielsweise Pseudomonas lachrymans;
Pyricularia-Arten, wie beispielsweise Pyricularia oryzae;
Pellicularia-Arten, wie beispielsweise Pellicularia sasakii, und
Pyrenophora-Arten, wie beispielsweise Pyrenophora teres (Konidienform: Drechslera, Synonym: Helminthosporium),
Leptosphaeria-Arten, wie beispielsweise Leptosphaeria nodorum;
Cochliobolus-Arten, wie beispielsweise Cochliobolus sativus, (Konidienform: Drechslera, Synonym: Helminthosporium);
Cercospora-Arten, wie beispielsweise Cercospora canescens.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die Verbindungen sind insbesondere wirksam gegen Cochliobolus sativus an Gerste, Fusarium culmorum an Weizen, Venturia in Apfel- und Pyricularia in Reiskulturen. Außerdem ist eine breite Wirkung als Spritzmittel und Saatbeizmittel gegen Getreidekrankheiten wie Mehltau, Rost, Pseudocercosporella herpotrichoides, Pyrenophera teres, Drechslera graminea und Fusarium nivale zu erwähnen.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol oder Alkylnaphtaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid; als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z. B. nichtionogene und anionische Emulgatoren, wie Polyoxyethylen-Fettsäure-Ester, Polyoxyethylen-Fettalkohol-Ether, z. B. Alkylarylpolyglykol-Ether, Alkylsulfonate Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewenden werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Herstellungsbeispiele:

Beispiel 1

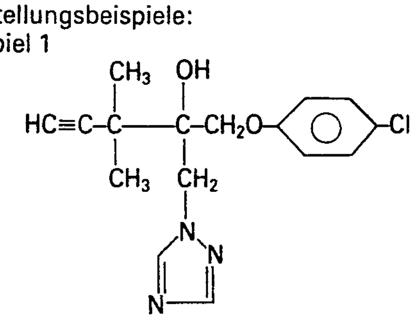

Zu einer siedenden Lösung von 5 g (0,052 Mol) Natriumbutanolat und 133 g (0,583 Mol) 1, 2, 4-Triazol in 300 ml n-Butanol werden unter Rühren 133 g (0,53 Mol) rohes 2-(4-Chlorphenoxymethyl)-2-(3-methyl-1-butin-3-yl)-oxiran, gelöst in 100 ml n-Butanol, zugetropft. Die Lösung wird während neun Stunden zum Sieden erhitzt. Anschließend wird das Lösungsmittel im Vakuum abdestilliert und der verbleibende ölige Rückstand in 500 ml Dichlormethan aufgenommen. Die Lösung wird dreimal mit je 1000 ml Wasser gewaschen, die organische Phase über Natriumsulfat getrocknet und anschließend im Vakuum eingedampft. Das zurückbleibende Öl (150 g) wird über Kieselgel mit Trichlormethan als Elutionsmittel filtriert. Man erhält so 101 g (0,31 Mol, das sind 58,5% der Theorie) 2-(4-Chlorphenoxymethyl)-3,3-dimethyl-1-(1, 2, 4-triazol-1-yl)-pent-4-in-2-ol als farblose Kristalle vom Schmelzpunkt 89–91 °C.

Daneben werden durch Verwendung von Essigsäureethylester als Elutionsmittel noch 20,6 g (das sind 11,9% der Theorie) 2-(4-Chlorphenoxymethyl)-3,3-dimethyl-1-(1, 2, 4-triazol-4-yl)-pent-4-in-2-ol als farblose Kristalle vom Schmelzpunkt 136–137 °C erhalten.

Vorprodukt 1:

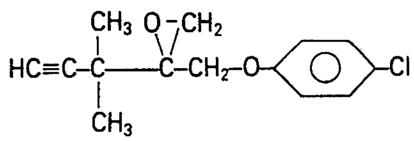

Zu einer Lösung von 56,9 ml (1,32 Mol) Dimethylsulfid in 200 ml tert.-Butanol werden zwischen 20 und 30 °C 114,5 ml (1,2 Mol) Dimethylsulfat zugetropft. Nach 12 Stunden werden 149 g (0,6 Mol) 1-(4-Chlorphenoxy)-3,3-dimethyl-4-pentin-2-on zugegeben und anschließend portionsweise 134,4 g (2,4 Mol) gepulvertes Kaliumhydroxid eingetragen. Man läßt das Gemisch 10 Stunden bei Raumtemperatur stehen und versetzt dann mit 1000 ml Wasser und 400 ml Toluol. Man trennt die organische Phase ab, extrahiert die wäßrige Phase zweimal mit je 200 ml Toluol, wäscht die vereinigten Toluol-Phasen dreimal mit jeweils 1000 ml Wasser, trocknet über Natriumsulfat und dampft im Vakuum ein. Es verbleiben 149 g (das sind 99% der Theorie) rohes 2-(4-Chlorphenoxymethyl)-2-(3-methyl-1-butin-3-yl)-oxiran als bräunliches, viskoses Öl, das ohne weitere Reinigung weiter umgesetzt werden kann.

Vorprodukt 2:

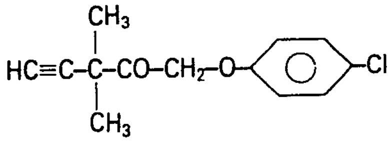

Zu einem siedenden Gemisch aus 86,1 g (0,67 Mol) 4-Chlorphenol und 92,5 g (0,67 Mol) gepulvertem Kaliumcarbonat in 500 ml Aceton wird unter Rühren eine Lösung von 98 g (0,67 Mol) 1-Chlor-3,3-dimethyl-4-pentin-2-on zugetropft. Nach fünf Stunden wird filtriert, das Filtrat eingedampft, der Rückstand in 250 ml Dichlormethan aufgenommen und mit 200 ml 1n Natronlauge und 200 ml Wasser gewaschen. Nach dem Abdestillieren des Lösungsmittels erhält man 149 g (das sind 94% der Theorie) 1-(4-Chlorphenoxy)-3,3-dimethyl-4-pentin-2-on als farbloses viskoses Öl, das beim Stehen kristallinisch erstarrt.

Vorprodukt 3:

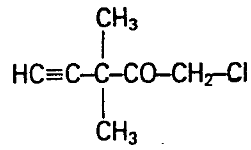

275 g (1,25 Mol) 1-Chlor-3,3-dimethyl-2-phenoxy-1-penten-4-in werden in 1,25 l Ethanol und 620 ml konzentrierter Salzsäure 5 Stunden unter Rückfluß erhitzt. Man verdünnt mit Wasser und extrahiert mehrfach mit n-Hexan. Nach Trocknen und Abziehen des Lösungsmittels bleiben 176 g Rohprodukt zurück. Durch Destillation erhält man 149,9 g (das sind 83% der Theorie) 1-Chlor-3,3-dimethyl-4-pentin-2-on vom Siedepunkt 67–70 °C/20 mm Hg.

NMR ($CDCl_3$): 1,45 (s, 6H); 2,5 (s, 1H); 4,75 (s, 2H).

Vorprodukt 4:

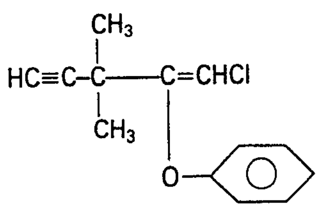

Methode A:

23,2 g (0,2 Mol) Natriumphenolat und 12,65 g (0,1 Mol) 1-Chlor-3,3-dimethyl-1,4-pentadiin werden in 100 ml absolutem N,N-Dimethylformamid 7 Stunden unter Rückfluß erhitzt. Man verdünnt mit Wasser und extrahiert mehrfach mit Dichlormethan, das mit verdünnter Natronlauge gewaschen wird. Nach Trocknen mit Abziehen des Lösungsmittels erhält man 16,4 g (das sind 74% der Theorie) 1-Chlor-3,3-dimethyl-2-phenoxy-1-penten-4-in vom Siedepunkt 85–95 °C/0,1 mm Hg.

NMR ($CDCl_3$): 1,45 (s, 6H); 2,3 (s, 1H); 6,45 (s, 1H); 6,7–7,5 (m, 5H).

Methode B:

163 g (1 Mol) 1,5-Dichlor-3,3-dimethyl-1-penten-4-in und 232 g (2 Mol) Natriumphenolat werden in 1 l absolutem N,N-Dimethylformamid innerhalb von 3 Stunden auf 140 °C erhitzt und 4 Stunden nachgerührt. Man arbeitet wie bei Methode A beschrieben auf und erhält so 204 g Rohprodukt, das nach der Destillation ($Kp._{0,15}$/87–94 °C) 141 g (das sind 64% der Theorie) 1-Chlor-3,3-dimethyl-2-phenoxy-1-penten-4-in liefert.

Vorprodukte 5 und 6

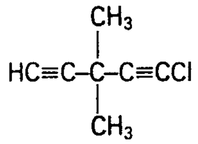

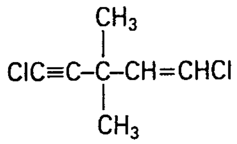

Eine Lösung von 800 g (20 Mol) NaOH in 200 ml Wasser wird auf 100 °C erwärmt. Unter Rühren werden 472 g (2 Mol) 1,1,5,5-Tetrachlor-3,3-dimethyl-1-penten und eine Lösung von 5 g Tetrabutylammoniumbromid in 3 ml Wasser zugegeben. Im Abstand von 4 Stunden werden viermal jeweils 5 g Tetrabutylammoniumbromid gelöst in 3 ml Wasser nachgegeben. Nach 20 Stunden wird das Reaktionsprodukt durch Wasserdampfdestillation aus dem Reaktionsgemisch abgetrennt. Das nichtwäßrige Destillat und die Wasserphase werden getrennt und anschließend die Reaktionsprodukte durch fraktionierte Kolonnendestillation im Vakuum rein isoliert. Man erhält so 12,9 g (4% der Theorie) 1,5-Dichlor-3,3-dimethyl-1-penten-4-in vom Siedepunkt 20 °C/18 mm Hg und 233 g (das sind 92% der Theorie) 1-Chlor-3,3-dimethyl-1,4-pentadiin vom Siedepunkt 50–52 °C/18 mm Hg.

Beispiel 2

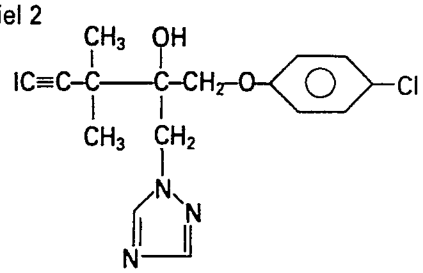

Zu einer Lösung von 4,8 g (0,015 Mol) 2-(4-Chlorphenoxymethyl)-3,3-dimethyl-1-(1,2,4-triazol-1-yl)-4-pentin-2-ol (siehe Beispiel 1) in 50 ml Methanol werden im Temperaturbereich zwischen 20 und 30 °C gleichzeitig 3,8 g (0,015 Mol) Jod und 7,2 ml (enthaltend 0,072 Mol Natriumhydroxid) konzentrierte Natronlauge eingerührt. Nach einer Stunde wird das Reaktionsgemisch in 500 ml Wasser eingerührt. Man extrahiert mit Dichlormethan, wäscht die organische Phase mit Wasser, trocknet über Natriumsulfat und engt im Vakuum ein. Der viskose Rückstand wird in Diethylether aufgenommen und die Lösung mit Petrolether bis zur beginnenden Trübung versetzt. Durch Anreiben erhält man 4,0 g (das sind 59,3% der Theorie) an 2-(4-Chlorphenoxymethyl)-3,3-dimethyl-5-jod-1-(1,2,4-triazol-1-yl)-4-pentin-2-ol vom Schmelzpunkt 108–109 °C.

In entsprechender Weise, wie in Beispiel 1 und 2 angegeben, werden die nachfolgenden Verbindungen der allgemeinen Formel (I)

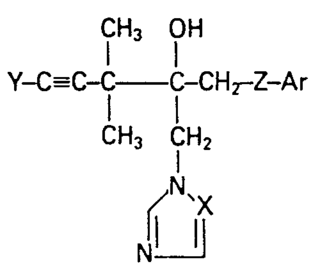 (I)

erhalten:

| Bsp. Nr. | X | Y | Z | Ar | Physik. Eigenschaften (Fp °C) |
|---|---|---|---|---|---|
| 3 | N | H | O | 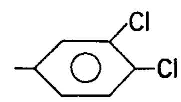 | 144–145 |
| 4 | N | H | O | 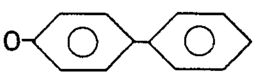 | Öl |
| 5 | N | H | O | 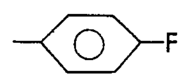 | 112–113 |
| 6 | N | H | O | 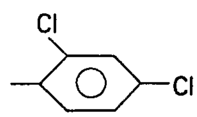 | 117–118 |
| 7 | N | H | O | 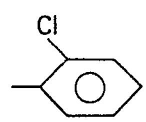 | 79–80 |
| 8 | N | H | O | 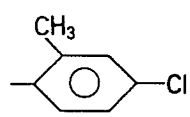 | Öl |
| 9 | N | I | O | 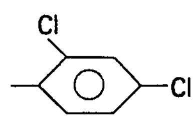 | Öl |
| 10 | CH | H | O | 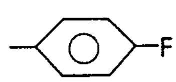 | 97–98 |
| 11 | N | I | O | 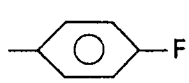 | 128–130 |
| 12 | CH | H | O | 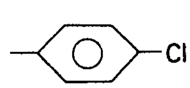 | 111–112 |
| 13 | CH | H | O | 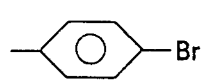 | 128–129 |
| 14 | N | H | O | 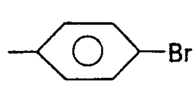 | 105–106 |

| Bsp. Nr. | X | Y | Z | Ar | Physik. Eigen-schaften (Fp °C) |
|---|---|---|---|---|---|
| 15 | CH | H | O | 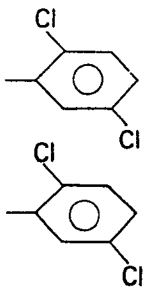 | 139–140 |
| 16 | N | H | O | | 133 |
| 17 | CH | H | O | 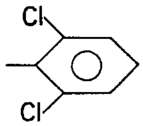 | Öl |
| 18 | N | H | O | 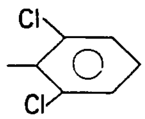 | Öl |
| 19 | CH | I | O | 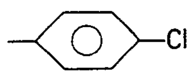 | Öl |
| 20 | CH | I | O | 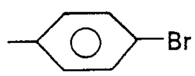 | Öl |
| 21 | N | I | O | 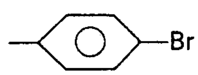 | 109–110 |
| 22 | CH | I | O | 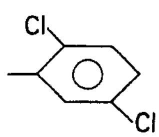 | Öl |
| 23 | N | I | O | 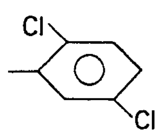 | Öl |
| 24 | N | I | O | 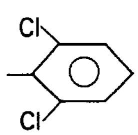 | Öl |

Zur Charakterisierung derjenigen erfindungsgemäßen Stoffe, die als Öle erhalten werden, dienen die nachstehend aufgeführten NMR-spektroskopischen Daten: ($^1H$–NMR); ppm

(Bsp. 4): ($CDCl_3$; 60 MHz)
1,45 (s, 6H); 2,3 (s, 1H); 3,95 (q, 2H); 4,1 (s, 1H); 4,68 (q, 2H); um 6,8 (m, 2H); um 7,4 (m, 7H); 7,9 (s, 1H); 8,15 (s, 1H).

(Bsp. 8): ($CDCl_3$; 60 MHz)
1,45 (s, 6H); 2,2 (s, 3H); 2,32 (s, 1H); um 3,9 (q, 2H); 4,18 (s, 1H); 4,68 (q, 2H); 6,5–7,2 (3H; m); 7,9 (s, 1H); 8,15 (s, 1H).

(Bsp. 9): ($CDCl_3$; 60 MHz)
1,48 (s, 6H); 3,9 (q, 2H); 4,1 (s, 1H); 4,75 (q, 2H); 6,6–7,45 (m, 3H); 7,9 (s, 1H); 8,22 (s, 1H).

(Bsp. 17): ($CDCl_3$; 80 MHz)
1,4 (d, 6H); 2,35 (s, 1H); um 4,2 (q, 2H); 4,2 (br. s, 1H); 4,9 (s, 2H); 6, 9–7, 7 (m, 6H).

(Bsp. 18): ($CDCl_3$; 80 MHz)
1,45 (d, 6H); 2,32 (s, 1H); um 4,2 (q, 2H); 4,1 (br. s, 1H); 4,7 (s, 2H); 6, 8–7, 4 (m, 3H); 7, 9 (s, 1H); 8,35 (s, 1H).

(Bsp. 19): ($CDCl_3$; 60 MHz)
1,45 (s, 6H); um 3,8 (q, 2H); 4, 1–4, 7 (m, 3H); 6, 5–7, 5 (m, 7H).

(Bsp. 20): ($CDCl_3$; 60 MHz)
1,45 (s, 6H); 3–4, 5 (m; 5H); 6, 65–6, 75 (m, 7H).

(Bsp. 22): ($CDCl_3$; 60 MHz)
1,5 (s, 6H); um 3,8 (q, 2H); 3,6 (br. s, 1H); um 4,45 (q, 2H); 6, 7–7, 7 (m, 6H).

(Bsp. 23): ($CDCl_3$; 60 MHz)
1,48 (s, 6H); um 3,85 (q, 2H); 4,1 (s, 1H); um 4,75 (q, 2H); 6, 7–7, 4 (m, 3H); 7,95 (s, 1H); 8,2 (s, 1H).

(Bsp. 24): ($CDCl_3$; 60 MHz)
1,45 (d, 6H); um 4,25 (q, 2H); um 4,1 (m, 1H); 4,7 (s; 2H); 6, 85–7, 6 (m, 3H); 7,95 (s, 1H); 8,35 (s, 1H).

Verwendungsbeispiele

In den nachfolgenden Verwendungsbeispielen wurden die folgenden Verbindungen als Vergleichssubstanzen eingesetzt:

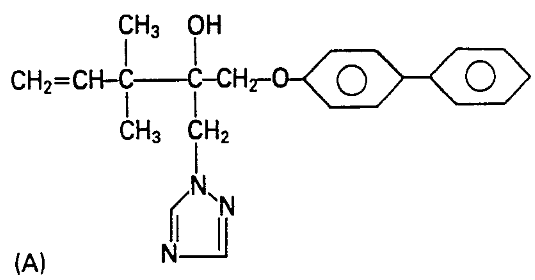
(A)

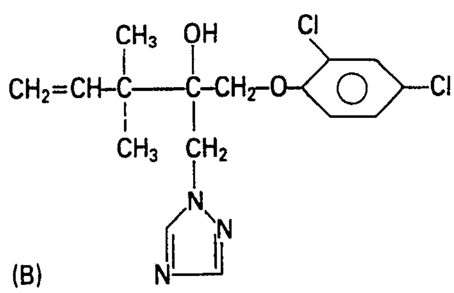
(B)

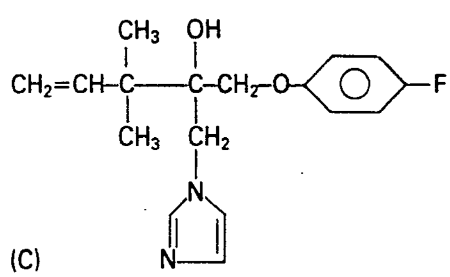
(C)

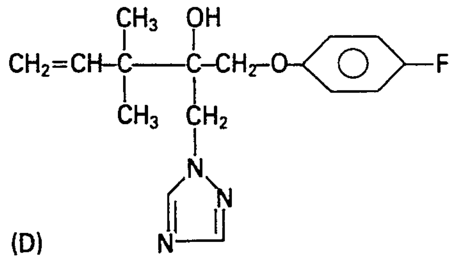

(D)

Beispiel A

Cochliobolus sativus-Test (Gerste)/protektiv

Lösungsmittel: 100 Gewichtsteile Dimethylformamid

Emulgator: 0,25 Gewichtsteile Alkylarylpolyglykolether.

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Cochliobolus sativus besprüht. Die Pflanzen verbleiben 48 Stunden bei 20°C und 100% rel. Luftfeuchtigkeit in einer Inkubationskabine.

Die Pflanzen werden in einem Gewächshaus bei einer Temperatur von 20°C und einer relativen Luftfeuchtigkeit von ca. 80% aufgestellt.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele: 1.

Beispiel B

Fusarium culmorum-Test (Weizen)/Saatgutbehandlung

Die Anwendung der Wirkstoffe erfolgt als Trokkenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das infizierte Saatgut 3 Minuten lang mit dem Beizmittel in einer verschlossenen Glasflasche.

Den Weizen sät man mit 2 × 100 Korn 1 cm tief in eine Standarderde und kultiviert ihn im Gewächshaus bei einer Temperatur von ca. 18°C in Saatkästen, die täglich 15 Stunden dem Licht ausgesetzt werden.

Ca. 3 Wochen nach der Aussaat erfolgt die Auswertung der Pflanzen auf Symptome.

Eine deutliche Überlegenheit in der Wirksamkeit gegenüber dem Stand der Technik zeigen bei diesem Test z.B. die Verbindungen gemäß folgender Herstellungsbeispiele: 3, 4, 5, 2, 1.

Beispiel C

Venturia-Test (Apfel)/protektiv

Lösungsmittel: 4,7 Gewichtsteile Aceton

Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20°C und 100% relativer Luftfeuchtigkeit in einer Inkubationskabine.

Die Pflanzen werden dann im Gewächshaus bei 20°C und einer relativen Luftfeuchtigkeit von ca. 70% aufgestellt.

12 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 1, 3, 4, 5, 2.

Beispiel D

Pyricularia-Test (Reis)/protektiv

Lösungsmittel: 12,5 Gewichtsteile Aceton

Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100% rel. Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß folgender Herstellungsbeispiele: 4, 1, 2, 6, 7.

Beispiel E

Pyricularia-Test (Reis)/systemisch

Lösungsmittel: 12,5 Gewichtsteile Aceton

Emulgator: 0,3 Gewichtsteile Alkylarylpolyglykolether

Zur Herstellung einer zweckmässigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25 °C und einer rel. Luftfeuchtigkeit von 100% bis zur Auswertung.

4 Tage nach der Inokulation erfolgt die Auswertung des Krankheitsbefalls.

Eine deutliche Überlegenheit in der Wirksamkeit gegenüber dem Stand der Technik zeigen in diesem Test z. B. die Verbindungen gemäß folgender Herstellungsbeispiele: 1, 5, 2.

**Patentansprüche**

1. Azolyl-aroxymethyl-dimethylpentinole der allgemeinen Formel (I)

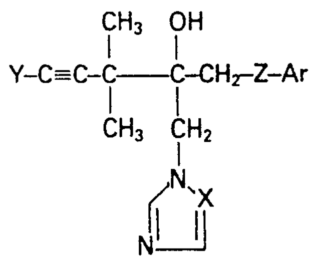

(I)

in welcher

X für ein Stickstoffatom oder die CH-Gruppe steht,

Y für ein Wasserstoff-, Brom- oder Jodatom steht,

Z für ein Sauerstoff- oder Schwefelatom, die $-SO-$ oder die $-SO_2$-Gruppe steht, und

Ar für Phenyl oder für Naphthyl steht, wobei die genannten Reste substituiert sein können durch Halogen, durch Alkyl, Alkoxy und Alkylthio mit jeweils bis zu 4 Kohlenstoffatomen, durch Halogenalkyl, Halogenalkoxy und Halogenalkylthio mit jeweils bis zu 2 Kohlenstoff- und bis zu 5 Halogenatomen, durch gegebenenfalls halogensubstituiertes Phenyl oder Phenoxy und schließlich durch die Gruppen $CH_3-O-N=CH-$ und $CH_3-O-N=C(CH_3)-$, sowie deren pflanzenphysiologisch verträgliche Säureadditions-Salze und Metallsalz-Komplexe.

2. Verfahren zur Herstellung von Azolyl-aroxymethyldimethylpentinolen der allgemeinen Formel (I)

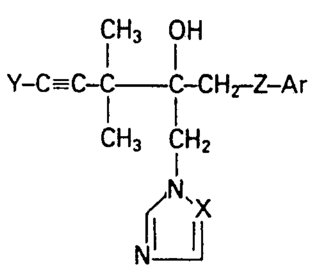

(I)

in welcher

X für ein Stickstoffatom oder die CH-Gruppe steht,

Y für ein Wasserstoff-, Brom- oder Jodatom steht,

Z für ein Sauerstoff- oder Schwefelatom, die $-SO-$ oder die $-SO_2$-Gruppe steht, und

Ar für Phenyl oder für Naphthyl steht, wobei die genannten Reste substituiert sein können durch Halogen, durch Alkyl, Alkoxy und Alkylthio mit jeweils bis zu 4 Kohlenstoffatomen, durch Halogenalkyl, Halogenalkoxy und Halogenalkylthio mit jeweils bis zu 2 Kohlenstoff- und bis zu 5 Halogenatomen, durch gegebenenfalls halogensubstituiertes Phenyl oder Phenoxy und schließlich durch die Gruppen $CH_3-O-N=CH-$ und $CH_3-O-N=C(CH_3)-$, sowie von deren pflanzenphysiologisch verträglichen Säureadditions-Salzen und Metallsalz-Komplexen, dadurch gekennzeichnet, daß man Oxiran-Derivate der allgemeinen Formel (II)

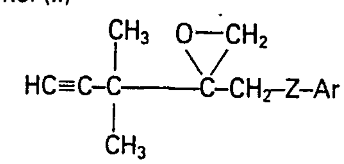

(II)

in welcher

Z und Ar die obengenannte Bedeutung besitzen, mit Azolen der allgemeinen Formel (III)

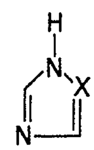

(III)

in welcher

X die obengenannte Bedeutung hat, gegebenenfalls in Gegenwart eines Verdünnungsmittels und einer Base zur Umsetzung bringt, und gegebenenfalls die dabei erhaltenen Verbindungen der allgemeinen Formel (Ia)

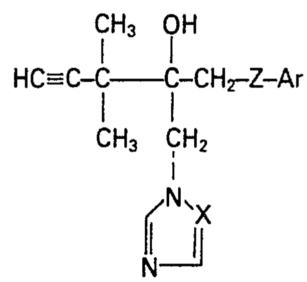

(Ia)

in welcher

X, Z und Ar die obengenannte Bedeutung besitzen, mit Brom oder Jod, gegebenenfalls in Gegenwart eines Verdünnungsmittels und eines Säurebindemittels, halogeniert, und weiterhin gegebenenfalls noch an die so erhaltenen Verbindungen der allgemeinen Formel (I) eine Säure oder ein Metallsalz addiert.

3. Fungizide Mittel, gekennzeichnet durch einen Gehalt an mindestens einem Azolyl-aroxymethyldimethylpentinol der allgemeinen Formel (I) gemäß Anspruch 1 bzw. eines pflanzenphysiologisch verträglichen Säureadditions-Salzes oder Metallsalz-Komplexes einer Verbindung der allgemeinen Formel (I).

4. Verfahren zur Bekämpfung von Pilzen, dadurch gekennzeichnet, daß man Azolyl-aroxymethyl-dimethylpentinole der allgemeinen Formel (I) gemäß Anspruch 1 bzw. deren pflanzenphysiologisch verträgliche Säureadditions-Salze bzw. Metallsalz-Komplexe auf Pilze oder ihren Lebensraum einwirken läßt.

5. Verwendung von Azolyl-aroxymethyl-dimethylpentinolen der allgemeinen Formel (I) gemäss Anspruch 1 bzw. von deren pflanzenphysiologisch verträglichen Säureadditions-Salzen und Metallsalz-Komplexen als Pflanzenschutzmittel.

6. Verwendung von Azolyl-aroxymethyl-dimethylpentinolen der allgemeinen Formel (I) gemäß Anspruch 1 bzw. von deren pflanzenphysiologisch verträglichen Säureadditions-Salzen und Metallsalz-Komplexen zur Bekämpfung von Pilzen.

7. Verfahren zur Herstellung von fungiziden Mitteln, dadurch gekennzeichnet, daß man Azolyl-aroxymethyl-dimethylpentinole der allgemeinen Formel (I) gemäß Anspruch 1 bzw. deren pflanzenphysiologisch verträgliche Säureadditions-Salze oder Metallsalz-Komplexe mit Streckmitteln und/oder oberflächenaktiven Mitteln vermischt.

## Claims

1. Azolyl-aroxymethyl-dimethylpentinols of the general formula (I)

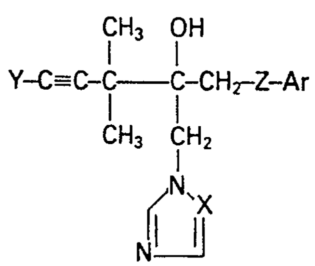

(I)

in which

X represents a nitrogen atom or the CH group,

Y represents a hydrogen, bromine or iodine atom,

Z represents an oxygen or sulphur atom, the –SO– group or the $-SO_2$-group and

Ar represents phenyl or naphthyl, it being possible for the said radicals to be substituted by halogen, by alkyl, alkoxy and alkylthio each with up to 4 carbon atoms, by halogenoalkyl, halogenoalkoxy and halogenoalkylthio each with up to 2 carbon atoms and up to 5 halogen atoms, by optionally halogensubstituted phenyl or phenoxy and, finally, by the groups $CH_3-O-N=CH-$ and $CH_3-O-N=C(CH_3)-$, and their phytophysiologically tolerated acid addition salts and metal salt complexes.

2. Process for the preparation of azolyl-aroxymethyl-dimethylpentinols of the general formula (I)

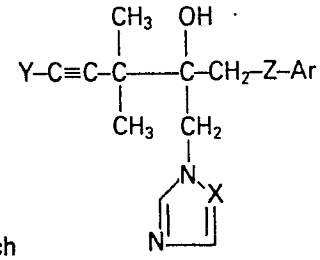

(I)

in which

X represents a nitrogen atom or the CH group,

Y represents a hydrogen, bromine or iodine atom,

Z represents an oxygen or sulphur atom, the –SO– group or the $-SO_2-$ group, and

Ar represents phenyl or naphthyl, it being possible for the said radicals to be substituted by halogen, by alkyl, alkoxy and alkylthio each with up to 4 carbon atoms, by halogenoalkyl, halogenoalkoxy and halogenoalkylthio each with up to 2 carbon atoms and up to 5 halogen atoms, by optionally halogen-substituted phenyl or phenoxy and, finally, by the groups $CH_3-O-N=CH-$ and $CH_3-O-N=C(CH_3)-$, and of their phytophysiologically tolerated acid addition salts and metal salt complexes, characterized in that oxirane derivatives of the general formula (II)

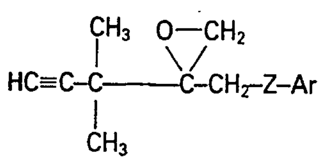

(II)

in which

Z and Ar have the abovementioned meaning, are reacted with azoles of the general formula (III)

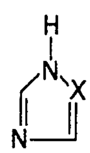

(III)

in which

X has the abovementioned meaning, if appropriate in the presence of a diluent and of a base and, if appropriate, the thus obtained compounds of the general formula (Ia)

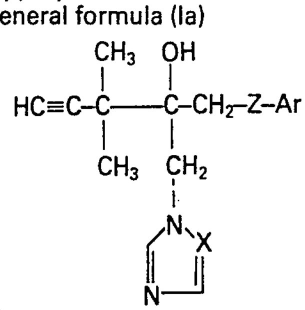

(Ia)

in which

X, Z and Ar have the abovementioned meaning, are halogenated with bromnine or iodine, if appropriate in the presence of a diluent and of an acid-binding agent, and furthermore, if appropriate, the thus resulting compounds of the general

formula (I) are subjected to an addition reaction on with an acid or a metal salt.

3. Fungicidal agents, characterized in that they contain at least one azolyl-aroxymethyl-dimethylpentinol of the general formula (I) according to claim 1 or a phytophysiologically tolerated acid addition salt or metal salt complex of a compound of the general formula (I).

4. Process for combating fungi, characterized in that azolyl-aroxymethyl-dimethylpentinols of the general formula (I) according to claim 1 or their phytophysiologically tolerated acid addition salts or metal salt complexes are allowed to act on fungi or their habitat.

5. Use of azolyl-aroxymethyl-dimethylpentinols of the general formula (I) according to claim 1 or their phytophysiologically tolerated acid addition salts and metal salt complexes as plant protection agents.

6. Use of azolyl-aroxymethyl-dimethylpentinols of the general formula (I) according to claim 1 or of their phytophysiologically tolerated acid addition salts and metal salt complexes for combating fungi.

7. Process for the preparation of fungicidal agents, characterized in that azolyl-aroxymethyl-dimethylpentinols of the general formula (I) according to claim 1 or their phytophysiologically tolerated acid addition salts or metal salt complexes are mixed with extenders and/or surfaceactive agents.

## Revendications

1. Azolyl-aroxyméthyl-diméthylpentynols de formule générale (I)

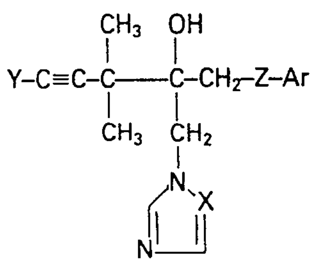

(I)

dans laquelle

X représente un atome d'azote ou le groupe CH,

Y représente un atome d'hydrogène, de brome ou d'iode,

Z représente un atome d'oxygène ou de soufre ou un groupe –SO– ou $-SO_2-$, et

Ar représente un radical phényle ou naphtyle, les restes nommés pouvant être substitués une ou plusieurs fois par un halogène, par un radical alkyle, alcoxy et alkylthio contenant chacun jusqu'à 4 atomes de carbone, par un radical halogéno-alkyle, halogéno-alcoxy et halogéno-alkylthio contenant chacun jusqu'à 2 atomes de carbone et jusqu'à 5 atomes d'halogène, par un radical phényle ou phénoxy éventuellement halogénosubstitué et enfin par les groupes $CH_3-O-N=CH-$ et $CH_3-O-N=C(CH_3)-$, ainsi que leurs sels d'addition d'acide et complexes de sels métalliques compatibles du point de vue de la physiologie végétale.

2. Procédé pour la fabrication des azolyl-aroxyméthyl-diméthylpentynols de formule générale (I)

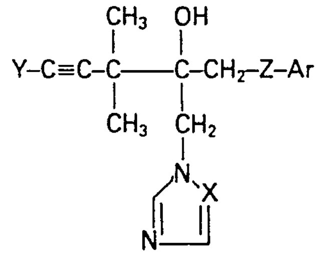

(I)

dans laquelle

X représente un atome d'azote ou le groupe CH,

Y représente un atome d'hydrogène, de brome ou d'iode,

Z représente un atome d'oxygène ou de soufre ou un groupe –SO– ou $-SO_2-$, et

Ar représente un radical phényle ou naphtyle, les restes nommés pouvant être substitués une ou plusieurs fois par un halogène, par un radical alkyle, alcoxy et alkylthio contenant chacun jusqu'à 4 atomes de carbone, par un radical halogéno-alkyle, halogéno-alcoxy et halogéno-alkylthio contenant chacun jusqu'à 2 atomes de carbone et jusqu'à 5 atomes d'halogène, par un radical phényle ou phénoxy éventuellement halogénosubstitué et enfin par les groupes $CH_3-O-N=CH-$ et $CH_3-O-N=C(CH_3)-$, ainsi que leurs sels d'addition d'acide et complexes de sels métalliques compatibles du point de vue de la physiologie végétale, caractérisé en ce que l'on fait réagir des dérivés oxirannés de formule générale (II)

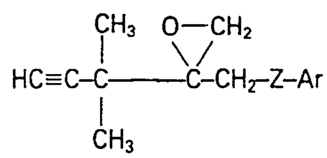

(II)

dans laquelle

Z et Ar possèdent les significations décrites ci-dessus, avec des azoles de formule générale (III)

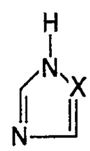

(III)

dans laquelle

X possède les significations décrites ci-dessus, éventuellement en présence d'un diluant et d'une base et éventuellement en halogénant les composés ainsi obtenus de formule générale (Ia)

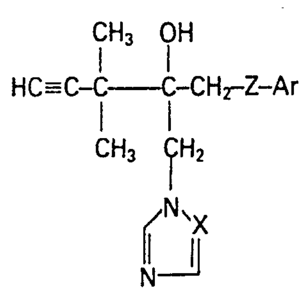

(Ia)

dans laquelle

X, Z et Ar ont les significations décrites ci-dessus, avec du brome ou de l'iode, éventuellement en présence d'un diluant et d'un fixateur d'acide, et en accolant éventuellement ensuite aux composés ainsi obtenus de formule générale (I) un acide ou un sel métallique.

3. Agents fongicides, caractérisé en ce qu'ils contiennent au moins un azolyl-aroxyléthyl-diméthylpentynol de formule générale (I) selon la revendication 1 ou un sel d'addition d'acide ou un complexe de sel métallique d'un composé de formule générale (I) physiologiquement compatible avec les végétaux.

4. Procédé pour la lutte contre les champignons, caractérisé en ce que l'on fait agir des azolylaromyméthyl-diméthylpentynols de formule générale (I) selon la revendication 1 ou leurs sels d'addition d'acide ou leurs complexes de sels métalliques physiologiquement compatibles avec les végétaux sur les champignons ou sur leur habitat.

5. Utilisation des azolyl-aroxyméthyl-diméthylpentynols de formule générale (I) selon la revendication 1 ou de leurs sels d'addition d'acide ou leurs complexes de sels métalliques physiologiquement compatibles avec les végétaux comme produits de protection des plantes.

6. Utilisation des azolyl-aroxyméthyl-diméthylpentynols de formule générale (I) selon la revendication 1 ou de leurs sels d'addition d'acide ou leurs complexes de sels métalliques physiologiquement compatibles avec les végétaux pour la lutte contre les champignons.

7. Procédé pour la fabrication de produits fongicides, caractérisé en ce qu'on mélange des azolylaroxyméthyl-diméthylpentynols de formule générale (I) selon la revendication 1 ou leurs sels d'addition d'acide ou leurs complexes de sels métalliques physiologiquement compatibles avec les végétaux avec des agents d'allongement ou diluants et/ou des agents tensio-actifs.